(19) Europäisches Patentamt
European Patent Office
Office européen des brevets

(11) Veröffentlichungsnummer: **0 149 238**
**B1**

(12) # EUROPÄISCHE PATENTSCHRIFT

(45) Veröffentlichungstag der Patentschrifft: **28.02.90**

(21) Anmeldenummer: **84116322.3**

(22) Anmeldetag: **27.12.84**

(51) Int. Cl. 5: C 07 D 239/26, C 07 D 239/34, C 09 K 19/34

(54) **Stickstoffhaltige Heterocyclen.**

(30) Priorität: **17.01.84 DE 3401321**

(43) Veröffentlichungstag der Anmeldung: **24.07.85 Patentblatt 85/30**

(45) Bekanntmachung des Hinweises auf die Patenterteilung: **28.02.90 Patentblatt 90/09**

(84) Bennante Vertragsstaaten: **CH DE FR GB LI**

(56) Entgegenhaltungen:
EP-A-0 056 501
EP-A-0 107 759
EP-A-0 152 697
GB-A-2 092 169
JP-A-54 041 285

PATENTS ABSTRACTS OF JAPAN, Band 6, Nr. 78 (C-102) 956, 15. Mai 1982; & JP - A - 57 14 584

**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Eidenschink, Rudolf, Dr.**
**Kornblumenstrasse 1**
**D-6115 Münster (DE)**
Erfinder: **Krause, Joachim, Dr.**
**Samuel-Morse-Strasse 14**
**D-6110 Dieburg (DE)**
Erfinder: **Hittich, Reinhard, Dr.**
**Am Kirchberg 11**
**D-6101 Modautal 1 (DE)**
Erfinder: **Poetsch, Eike, Dr.**
**Am Buchwald 4**
**D-6109 Mühltal 6 (DE)**
Erfinder: **Scheuble, Bernhard, Dr.**
**Am Grenzweg 18**
**D-6146 Alsbach (DE)**
Erfinder: **Weber, Georg**
**Wilhelm-Leuschnerstrasse 38**
**D-6106 Erzhausen (DE)**
Erfinder: **Pohl, Ludwig, Dr.**
**Niebergallweg 5**
**D-6100 Darmstadt (DE)**

EP 0 149 238 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft stickstoffhaltige Heterocyclen der Formel I

$R^1$-(A-Z)$_n$-A-$R^2$ I

worin

| | |
|---|---|
| $R^1$ und $R^2$ | jeweils unabhängig voneinander eine Alkylgruppe mit 1 - 15 C-Atomen, worin auch eine oder zwei nicht benachbarte $CH_2$-Gruppen durch O-Atome und/oder -CO-Gruppen und/oder -O-CO-Gruppen und/oder -CO-O-Gruppen ersetzt sein können, einer der Reste $R^1$ und $R^2$ auch H, CN, F, Cl oder Br, |
| die Gruppen A | jeweils unabhängig voneinander eine 1,4-Phenylengruppe, worin auch eine oder zwei CH-Gruppen durch N-Atome ersetzt sein können, eine 1,4-Cyclohexylengruppe, worin auch eine oder zwei nicht benachbarte $CH_2$-Gruppen durch O-Atome ersetzt sein können, eine 1,3-Dithian-2,5-diyl-Gruppe, eine 1,4-Bicyclo(2,2,2)-octylen-Gruppe, eine Decahydronaphthalin-2,6-diyl-Gruppe oder eine 1,2,3,4-Tetrahydronaphthalin-2,6-diylgruppe, |
| die Gruppen Z | jeweils unabhängig voneinander -CO-O-, -O-CO-, -$CH_2CH_2$-, -$OCH_2$- -$CH_2O$- oder eine Einfachbindung und |
| n | 3 oder 4 |

bedeutet, mit den Maßgaben, daß

(a) mindestens eine der Gruppen A eine 1,4-Phenylengruppe, worin zwei CH-Gruppen durch N-Atome ersetzt sind, bedeutet, und
(b) mindestens eine der weiteren Gruppen A einen cycloaliphatischen Rest enthält und/oder mindestens eine Gruppe Z nicht eine Einfachbindung ist.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine 1,4-Bicyclo(2,2,2)-octylengruppe, Phe eine 1,4-Phenylengruppe, Pyn eine Pyrimidin-2,5-diylgruppe und Pyn eine Pyridazin-3,6-diylgruppe.

Ähnliche Verbindungen sind z. B. in der JP-OS-54 041 285 genannt. Die dort angegebenen Verbindungen enthalten jedoch im Gegensatz zu den vorliegenden keine cycloaliphatischen Ringe (Cy, Dio, Dit, Bi) bzw. kein Brückenglied Z.

Weiterhin werden in der EP-0 056 501 und der GB-2 092 169 aus nur zwei aromatischen Ringen bestehende 4-(5-Alkylpyrimidin-2-yl)-benzonitrile als Mischungskomponenten für TN-Zellen beschrieben.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Güst-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplexraten herstellbar.

Überraschenderweise zeigte sich beim Zusatz von Verbindungen der Formel I zu flüssigkristallinen Phasen, daß selbst größere Zusätze (z. B. von 30 %) die Schwellenspannung nur unwesentlich erhöhen. Völlig unerwartet tritt gleichzeitig eine erhebliche Verbesserung der Kennliniensteilheit der Mischung auf, so daß Verbindungen des Typs I als besonders vorteilhaft geeignete Substanzen zur Herstellung von flüssig-kristallinen Mischungen mit steiler Kennlinie anzusehen sind. Sie ermöglichen damit die Entwicklung hoch multiplexbarer Mischungen sehr kleiner optischer Anisotropie, mit denen eine Drehzelle insbesondere im ersten Transmissionsminimum nach Gooch-Tarry betrieben werden kann. Damit ergibt sich eine sehr kleine Beobachtungswinkel-Abhängigkeit des Kontrastes.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich als Bestandteile flüssigkristalliner Dielektrika verwenden lassen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man eine Verbindung, die sonst der Formel I entspricht, aber an Stelle von C=C-Doppelbindungen, eine oder mehrere oxidierbare Gruppen wie -$CH_2CH_2$-, enthält, mit einem oxidierenden Mittel behandelt,

oder daß man zur Herstellung von Estern der Formel I (worin $R^1$ und/oder $R^2$ eine Alkylgruppe bedeuten, worin eine oder zwei $CH_2$-Gruppen durch -O-CO- Gruppen und/oder -CO-O-Gruppen ersetzt sind und/oder worin mindestens eine der Gruppen Z -CO-O- oder -O-CO- bedeutet) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von 1,3-Dioxanderivaten bzw. 1,3-Dithianderivaten der Formel I (worin A 1,3-Dioxan-2,5-diyl bzw. 1,3-Dithian-2,5-diyl bedeutet) einen entsprechenden Aldehyd mit einem entsprechenden Diol bzw. Dithiol umsetzt,

oder daß man zur Herstellung von Ethern der Formel I (worin $R^1$ und/oder $R^2$ eine Alkylgruppe bedeuten, worin eine oder zwei $CH_2$-Gruppen durch O-Atome ersetzt sind und/oder Z eine -$OCH_2$- oder -$CH_2O$-Gruppe ist) eine entsprechende Hydroxyverbindung verethert,

und/oder daß man gegebenenfalls eine Base der Formel I durch Behandeln mit einer Säure in eines ihrer Säureadditionssalze umwandelt,

oder daß man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Vor- und nachstehend haben $R^1$, $R^2$, A und Z die angegebene Bedeutung, sofern nicht ausdrücklich etwas anderes vermerkt ist.

Die Verbindungen der Formel I umfassen dementsprechend bevorzugte Verbindungen der Teilformeln Ia bis In (ohne Brückenglieder Z). Der Einfachheit halber bedeuten im folgenden $A^1$ eine 1,4-Phenylengruppe, worin zwei CH-Gruppen durch N-Atome ersetzt sind, $A^2$ eine cycloaliphatische Gruppe A und p 2 oder 3.

| | |
|---|---|
| $R^1$-$A^1$-$(A)_p$-$A^2$-$R^2$ | Ia |
| $R^1$-$A^1$-$(A)_{p-1}$-$A^2$-A-$R^2$ | Ib |
| $R^1$-$A^1$-$(A)_{p-2}$-$A^2$-A-A-$R^2$ | Ic |
| $R^1$-$A^1$-$A^2$-A-A-$R^2$ | Id |
| $R^1$-$A^1$-$A^2$-A-A-A-$R^2$ | Ie |
| $R^1$-A-$A^1$-$(A)_{p-1}$-$A^2$-$R^2$ | If |
| $R^1$-$A^2$-$A^1$-$(A)_p$-$R^2$ | Ig |
| $R^1$-A-$A^1$-$(A)_{p-2}$-$A^2$-A-$R^2$ | Ih |
| $R^1$-A-$A^1$-$A^2$-$(A)_{p-1}$-$R_2$ | Ii |
| $R^1$-A-A-$A^1$-$(A)_{p-2}$-$A^2$-$R^2$ | Ij |
| $R^1$-A-A-$A^1$-$A^2$-$R^2$ | Ik |
| $R^1$-A-A-$A^1$-$A^2$-A-$R^2$ | Il |
| $R^1$-$A^2$-A-$A^1$-$(A)_{p-1}$-$R^2$ | Im |
| $R^1$-A-$A^2$-$A^1$-$(A)_{p-1}$-$R^2$ | In |

Darunter sind diejenigen der Teilformeln 1a - 1h besonders bevorzugt.

Die Verbindungen der Formel I umfassen weiterhin bevorzugte Verbindungen der Teilformeln Io bis Iz mit einem Brückenglied Z.

| | |
|---|---|
| $R^1$-$A^1$-Z-$(A)_{p+1}$-$R^2$ | Io |
| $R^1$-$A^1$-A-Z-$(A)_p$-$R^2$ | Ip |
| $R^1$-$A^1$-A-A-Z-$(A)_{p-1}$-$R^2$ | Iq |
| $R^1$-$A^1$-A-A-A-Z-$(A)_{p-2}$-$R^2$ | Ir |
| $R^1$-A-Z-$A^1$-$(A)_p$-$R^2$ | Is |
| $R^1$-A-$A^1$-Z-$(A)_p$-$R^2$ | It |
| $R^1$-A-$A^1$-A-Z-$(A)_{p-1}$-$R^2$ | Iu |
| $R^1$-A-$A^1$-A-A-Z-$(A)_{p-2}$-$R^2$ | Iv |
| $R^1$-A-Z-A-$A^1$-$(A)_{p-1}$-$R^2$ | Iw |
| $R^1$-A-A-Z-$A^1$-$(A)_{p-1}$-$R^2$ | Ix |
| $R^1$-A-A-$A^1$-Z-$(A)_{p-1}$-$R^2$ | Iy |
| $R^1$-A-A-$A^1$-A-Z-$(A)_{p-2}$-$R^2$ | Iz |

Darunter sind diejenigen der Teilformeln Io - Iu besonders bevorzugt. Bevorzugte Verbindungen der Teilformel It sind diejenigen der Teilformel Itl:

$R^1$-Phe-Pyr-COO-A-A-$R^2$ Itl

Die Verbindungen der Formel I umfassen ferner Verbindungen der Teilformeln I1 bis I5 (mit 2 Brückengliedern Z), I6 und I7 (mit 3 Brückengliedern Z) sowie I8 (mit 4 Brückengliedern Z).

| | |
|---|---|
| $R^1$-A-Z-A-Z-$(A)_{n-1}$-$R^2$ | I1 |
| $R^1$-A-Z-A-A-Z-$(A)_{n-2}$-$R^2$ | I2 |
| $R^1$-A-Z-A-A-A-Z-$(A)_{n-3}$-$R^2$ | I3 |
| $R^1$-A-A-Z-A-Z-$(A)_{n-2}$-$R^2$ | I4 |
| $R^1$-A-A-Z-A-A-Z-$(A)_{n-3}$-$R^2$ | I5 |
| $R^1$-A-Z-A-Z-A-Z-$(A)_{n-2}$-$R^2$ | I6 |
| $R^1$-A-Z-A-Z-A-A-Z-A-$R^2$ | I7 |
| $R^1$-A-Z-A-Z-A-Z-A-Z-A-$R^2$ | I8 |

Darunter sind diejenigen der Teilformeln I1 und I2 besonders bevorzugt.

In den Verbindungen der vor- und nachstehenden Formeln bedeuten $R^1$ und $R^2$ vorzugsweise Alkyl, Alkoxy oder eine andere Oxaalkylgruppe. Besonders bevorzugt sind weiterhin Verbindungen der vor- und nachstehenden Formeln worin einer Reste $R^1$ und $R^2$ Alkyl und der andere Rest Alkoxy oder CN bedeutet. Weiterhin bevorzugt sind Verbindungen der vorund nachstehenden Formeln, worin keiner der Reste $R^1$ und $R^2$ H bedeutet, sowie Verbindungen der vor- und nachstehenden Formeln, worin beide Reste $R^1$ und $R^2$ jeweils unabhängig voneinander n-Alkyl bedeuten.

Die Gruppe A sind bevorzugt Cy, Phe, $A^1$ oder $A^2$, ferner Dio oder Dit; bevorzugt enthält die Verbindung der Formel I nicht mehr als einen der Reste Dio oder Dit.

$A^1$ ist bevorzugt Pyr, Pyn oder Pyrazin-2,5-diyl, besonders bevorzugt Pyr.

Der cycloaliphatische Rest $A^2$ ist bevorzugt Cy, Dio, Dit oder Bi, besonders bevorzugt Cy oder Dio. Bevorzugte Verbindungen der Formel I enthalten eine oder zwei, insbesondere zwei, Gruppen $A^2$.

Z ist bevorzugt eine Einfachbindung, in zweiter Linie bevorzugt -CO-O-, -O-CO- oder $-CH_2CH_2-$.
n ist vorzugsweise 3.

Falls $R^1$ und/oder $R^2$ Alkylreste bedeuten, in denen auch eine ("Alkoxy" bzw. "Oxaalkyl") oder zwei ("Alkoxyalkoxy" bzw. "Dioxaalkyl") $CH_2$-Gruppen durch O-Atome ersetzt sein können, so können sie geradkettig oder verzweigt sein. Vorzugsweise sind sie geradkettig, haben 2, 3, 4 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl, Dodecyl, Ethoxy, Propoxy, Butoxy, Pentoxy, Hexoxy, Heptoxy, Octoxy, Nonoxy, Decoxy, Undecoxy, Dodecoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl), 2-, 3-, 4-, 5- oder 6-Oxaheptyl, ferner Methyl, Methoxy, Tridecyl, Tridecoxy, Tetradecyl, Tetradecoxy, Pentadecyl, Pentadecoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (=Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formeln I mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Octyloxy, 2-Oxa-3-methylbutyl, 3-Oxa-4-methylpentyl.

Unter den Verbindungen der vor- und nachstehenden Formeln I sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln I9 bis I15:

| | |
|---|---|
| $R^1$-$A^1$-A-$A^2$-$A^2$-$R^2$ | I9 |
| $R^1$-$A^2$-$A^1$-A-$A^2$-$R^2$ | I10 |
| $R^1$-$A^2$-$A^1$-$A^2$-$A^2$-$R^2$ | I11 |
| $R^1$-$A^1$-A-Z-A-$A^2$-$R^2$- | I12 |
| $R^1$-$A^1$-A-Z-$A^2$-$A^2$-$R^2$ | I13 |
| $R^1$-$A^2$-Z-$A^1$-A-Z-A-$R^2$ | I14 |
| $R^1$-$A^2$-Z-$A^1$-A-Z-$A^2$-$R^2$ | I15 |

Unter den Verbindungen der Formel I sind diejenigen Stereoisomeren bevorzugt, in denen die cycloaliphatischen Reste (z. B. Cy, Dio, Dit) trans-1,4-disubstitutiert sind.

Weiterhin bevorzugt sind diejenigen Verbindungen der Formel I, worin mindestens zwei der weiteren Gruppen A einen cycloliphatischen Rest $A^2$ bedeuten und mindestens eine Gruppe Z -CO-O-, -O-CO- oder $-CH_2CH_2-$ ist.

Diejenigen der vorstehend genannten Formeln, die eine oder mehrere der Gruppen Dio, Dit und/oder Pyr enthalten, umschließen jeweils die beiden möglichen 2,5-Stellungsisomeren.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen (z. B. DE-OS-2 344 732, 2 450 088, 2 429 093, 2 502 904, 2 636 684, 2 701 591 und 2 752 975 betreffend Verbindungen mit 1,4-Cyclohexylen- und 1,4-Phenylen-Gruppen; DE-PS-2 641 724 betreffend Verbindungen mit Pyrimidin-2,5-diyl-Gruppen; DE-OS-3 238 350 betreffend Verbindungen mit Pyridazin-2,5-diyl-Gruppen; JP-OS-5 843 961 betreffend Verbindungen mit Pyrazin-2,5-diyl-Gruppen; DE-OS-2 944 905 und 3 227 916 betreffend Verbindungen mit 1,3-Dioxan-2,5-diyl-Gruppen; DD 160 061 betreffend Verbindungen mit 1,3-Dithian-2,5-diyl-Gruppen; US-4 261 652 und 4 219 256 betreffend Verbindungen mit 1,4-Bicyclo-(2,2,2)-octylen-Gruppen; DE-OS-3 150 312 betreffend Verbindungen mit Decahydronaphthalin-2,6-diyl-Gruppen; DE-OS-2 949 080 betreffend Verbindungen mit 1,2,3,4-Tetrahydronapthalin-2,6-diyl-Gruppen; DE-OS-3 201 721 betreffend Verbindungen mit $-CH_2-CH_2-$Brückengliedern; DE-OS-2 139 628, DE-OS-2 535 046 und DE-OS-2 800 553 betreffend Verbindungen mit Z = -CO-O- oder -O-CO- und DE-OS-3 001 661, DE-OS-3 040 632 und DE-OS-3 149 139 betreffend Verbindungen mit Z = $-CH_2O-$ bzw. $-OCH_2-$).

Im allgemeinen wurden zwei Teilverbindungen (z. B. (1) und (2) (Schema 1) oder (3) und (4) (Schema 2)) zu Verbindungen der Formel I kondensiert:

**Schema 1**

$$R^1\text{-}(A\text{-}Z)_p\text{-}M^1L^1 + L^2M^2\text{-}Z\text{-}(A\text{-}Z)_q\text{-}A\text{-}R^2$$

(1) (2)

$$\downarrow -L^1L^2$$

$$R^1\text{-}(A\text{-}Z)_p\text{-}M^1M^1\text{-}Z\text{-}(A\text{-}Z)_q\text{-}A\text{-}R^2 \qquad \text{I}$$

$(M^1M^2 = A)$

$(p + q = n)$

**Schema 2**

$$R^1\text{-}(A\text{-}Z)_{p\text{-}1}\text{-}A\text{-}M^3L^3 + L^4M^4\text{-}(A\text{-}Z)_q\text{-}A\text{-}R^2$$

(3) (4)

$$\downarrow -L^3L^4$$

$$R^1\text{-}(A\text{-}Z)_{p\text{-}1}\text{-}A\text{-}M^3M^4\text{-}(A\text{-}Z)_q\text{-}A\text{-}R^2 \qquad \text{I}$$

$(M^3M^4 = Z)$

$(p + q = n)$

$-M^1L^1$ und $-M^2L^2$ sind kondensationsfähige Baustein die z. B. Malonsäurederivaten (z. B. Malondialdehyd), Amidinen, Aldehyden, 1,3-Propandiolen und/oder 1,3-Propandithiolen entsprechen. $L^1L^2$ sind eine oder mehrere abgespaltene Gruppen.

$-M^3L^4$ und $-M^4L^4$ sind kondensationsfähige Bausteine, z. B. ausgewählt aus der Gruppe -COOH, -CO-Halogen, -OH, -O-Metall, $-CH_2$-Halogen, $-CH_2$-Metall, $-CH_2-OH$, $-CH_2$-O-Metall, -Metall, -Halogen.

$L^3$ und $L^4$ sind Abgangsgruppen. $L^3L^4$ ist eine abgespaltene Gruppe wie z. B. $H_2O$, H-Halogen, Metall-Halogen.

Weiterhin können jedoch auch entsprechende intramolekulare Kondensationen zur Synthese von Verbindungen der Formel I durchgeführt werden (z. B. Kondensation von 1,4-Diketonen mit Hydrazin (z. B. DE-OS-3 238 350) oder Umsetzung eines Butadienderivates z. B. mit Acetylendicarbonsäurederivaten (z. B. JP-OS-58 144 327; JP-OS-58 146 543).

Die Ausgangsstoffe sind bekannt oder können nach analogen Methoden wie die bekannten Verbindungen erhalten werden. Der Fachmann kann durch Routinemethoden entsprechende Ausgangsstoffe und/oder Methoden zu deren Synthese aus dem Stand der Technik entnehmen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonyl-Gruppen in Betracht, insbesondere Ketogruppen, ferner z. B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer $-CH_2CH_2$-Gruppe eine -CH=CH-Gruppe und/oder an Stelle einer $-CH_2$-Gruppe eine -CO-Gruppe und/oder an Stelle eines H-Atoms eine freie oder eine funktionell (z. B. in Form ihres p-Toluolsulfonats) abgewandelte OH-Gruppe enthalten.

Die Reduktion kann z. B. erfolgen durch katalytische Hydrierung bei Temperaturen zwischen etwa 0° und etwa 200° sowie Drucken zwischen etwa 1 und 200 bar in einem inerten Lösungsmittel, z. B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z. B. $PtO_2$, PdO), auf einem Träger (z. B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200°) zu den entsprechenden Verbindungen der Formel I, die Alkylgruppen und/oder $-CH_2-CH_2$-Brücken enthalten, reduziert werden.

Weiterhin sind Reduktionen mit komplexen Hydriden möglich. Beispielsweise können Arylsulfonyloxygruppen mit $LiAlH_4$ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können (auch in Gegenwart von CN-Gruppen!) mit $NaBH_4$ oder Tributylzinnhydrid in Methanol hydriert werden; so entstehen z. B. aus 1-Cyancyclohexenderivaten die entsprechenden Cyclohexanderivate.

Verbindungen der Formel I sind weiterhin erhältlich, indem man eine Verbindung, die sonst der Formel I entspricht, aber an Stelle von C=C-Doppelbindungen, eine oder mehrere oxidierbare Gruppen wie $-CH_2-CH_2$-Gruppen enthält, oxidiert.

Pyridazine der Formel I sind beispielsweise durch Oxidation eines entsprechenden 4,5-Dihydropyridazins oder eines tautomeren Dihydropyridazins erhältlich.

Die Oxidation einer solchen Verbindung kann in an sich bekannter Weise z. B. mit 2,3-Dichlor-5,6-dicyan-p-benzochinon in Dioxan, mit Natriumnitrit in Eisessig und Äthanol, mit Isopentylnitrit in Eisessig und dergleichen durchgeführt werden. Temperatur und Druck sind nicht kritisch bei dieser Reaktion. Zweckmäßigerweise werden jedoch Atmosphärendruck und eine Temperatur zwischen Raumtemperatur und Rückflußtemperatur, vorzugsweise etwa Raumtemperatur, angewendet. Vorzugsweise werden die Ausgangs-Verbindungen jedoch durch katalytische Dehydrierung in an sich bekannter Weise zu Verbindungen der Formel I oxidiert. Die Dehydrierung kann mit irgendeinem in Dehydrierungsreaktionen üblicherweise verwendeten Katalysator, wie Palladium, Platin und dergleichen (gegebenenfalls auf einem inerten Trägermaterial, z. B. Kohle) durchgeführt werden. Bevorzugter Katalysator ist Palladium. Als Lösungsmittel können irgendwelche inerte organische Lösungsmittel, wie Alkohole, Ether, Ester, Carbonsäuren und dergleichen, beispielsweise Ethanol, Dioxan, Essigsäure-ethylester oder Eisessig verwendet werden. Bevorzugtes Lösungsmittel ist Ethanol. Temperatur und Druck sind nicht kritisch bei dieser Reaktion. Zweckmäßigerweise werden eine Temperatur zwischen Raumtemperatur und der Rückflußtemperatur des Reaktionsgemisches und Atmosphärendruck angewendet.

Die 4,5-Dihydropyridazine können durch Wanderung der Doppelbindungen im Dihydropyridazin-Ring zu tautomeren Verbindungen umlagern. Derartige Umlagerungen können z. B. durch Anwesenheit einer Spur Säure oder Base ausgelöst werden. Da die tautomeren Dihydropyridazine jedoch ebenfalls unter den obigen Bedingungen zu Verbindungen der Formel I oxidiert werden können, kann sowohl ein 4,5-Dihydropyridazin als auch ein tautomeres Dihydropyridazin oder ein Gemisch solcher Verbindungen umgesetzt werden. Die Dihydropyridazine sind durch Umsetzung entsprechender 1,4-Diketone mit Hydrazin zugänglich (DE-OS-3 228 350).

Ester der Formel I ($R^1$ und/oder $R^2$ = Alkyl, worin auch eine oder zwei $CH_2$-Gruppen durch -O-CO-Gruppen und/oder -CO-O-Gruppen ersetzt sind oder Z = -CO-O- oder -O-CO-) können auch durch Veresterung entsprechender Carbonsäuren (oder ihrer reaktionsfähigen Derivate) mit Alkoholen bzw. Phenolen (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1 - 4 C-Atomen in der Alkylgruppe.

Als reaktionsfähige Derivate der genannten Alkohole bzw. Phenole kommen insbesondere die entsprechenden

Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natriumoder Kaliumacetat, Erdalkalimetallhydroxide wie Calciumhydroxid oder organische Basen wie Triethylamin, Pyridi Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25° und +20°.

Dioxanderivate bzw. Dithianderivate der Formel I (worin eine der Gruppen A eine 1,3-Dioxan-2,5-diyl-Gruppe bzw. eine 1,3-Dithian-2,5-diyl-Gruppe bedeutet) werden zweckmäßig durch Reaktion eines entsprechenden Aldehyds mit einem entsprechenden 1,3-Diol bzw. einem entsprechenden 1,3-Dithiol (oder einem seiner reaktionsfähigen Derivate) hergestellt, vorzugsweise in Gegenwart eines inerten Lösungsmittels wie Benzol oder Toluol und/oder eines Katalysators, z. B. einer starken Säure wie Schwefelsäure, Benzol- oder p-Toluolsulfonsäure, bei Temperaturen zwischen etwa 20° und etwa 150°, vorzugsweise zwischen 80° und 120° Als reaktionsfähige Derivate der Ausgangsstoffe eignen sich in erster Linie Acetale.

Die genannten Aldehyde, 1,3-Diole und 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester, sowie die Dithiole durch Umsetzung entsprechender Dihalogenide mit NaSH erhältlich.

Zur Herstellung von Nitrilen der Formel I (worin $R^1$ oder $R^2$ CN bedeutet) können entsprechende Säureamide dehydratisiert werden. Die Amide sind z. B. aus entsprechenden Estern oder Säurehalogeniden durch Umsetzung mit Ammoniak erhältlich. Als wasserabspaltende Mittel eignen sich beispielsweise anorganische Säurechloride wie $SOCl_2$, $PCl_3$, $PCl_5$, $POCl_3$, $SO_2Cl_2$, $COCl_2$, ferner $P_2O_5$, $P_2S_5$, $AlCl_3$ (z. B. als Doppelverbindung mit NaCl), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z. B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel I kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80° und 150°, vorzugsweise bei 120°. Nach üblicher Aufarbeitung kann man direkt die Nitrile isolieren.

Ether der Formel I (worin $R^1$ und/oder $R^2$ eine Alkylgruppe bedeuten, worin eine oder zwei $CH_2$-Gruppen durch O-Atome ersetzt sind, und/oder worin mindestens ein Z eine $-OCH_2-$ oder eine $-CH_2O-$Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z. B. durch Behandeln mit NaH, $NaNH_2$, NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, 1,2-Dimethoxyethan, DMF oder Dimethylsulfoxid oder auch einem Überschuß an wäßriger oder wäßrig-alkoholischer NaOH oder KOH bei Temperaturen zwischen etwa 20° und 100°.

Eine Base der Formel I kann mit einer Säure in das zugehörige Säureadditionssalz übergeführt werden. Für diese Umsetzung können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z. B. mit einer starken anorganischen Base wie KOH oder NaOH.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexyl-ethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren,

R'-L-G-E-R" II

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| G | CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Phe-COO- |

oder eine C-C-Einfachbindung, Y Halogen, vorzugsweise Chlor, oder -CN, und R' und R" Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$ $CF_3$, F, Cl oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z. B. I. Haller et al., Mol. Cryst. Liq. Cryst. Band *24,* Seiten 249 - 258 (1973)) zur Verbesserung der Leitfähigkeit, dichroitische Farbstoffe zur Herstellung farbiger Güst-Host-Systeme oder Substanzen zur Veränderung der dielektrischen Anisotropie, der Viskosität und/oder der Orientierung der nematischen Phasen zugesetzt werden. Derartige Substanzen sind z. B. in den DE-OS-2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

**Beispiel 1**

Man erhitzt 3,2 g n-Heptylmalondialdehydtetraethylacetal, 3,9 g 4-(trans,trans-4-n-Pentylbicyclohexy-4'-yl)benzamidin-

hydrochlorid und 5 ml Dimethylformamid (DMF) 12 Stunden auf 150°.

Übliche Aufarbeitung liefert 4 g 2-[4-(trans,trans-4-n-Pentylbicyclohex-4'-yl)-phenyl]-5-n-heptylpyrimidin.

Analog werden hergestellt:

2-[4-(trans,trans-4-Pentylbicyclohex-4'-yl)phenyl]-5-ethylpyrimidin
2-[4-(trans,trans-4-Pentylbicyclohex-4'-yl)phenyl]-5-propylpyrimidin
2-[4-(trans,trans-4-Pentylbicyclohex-4'-yl)phenyl]-5-butylpyrimidin
2-[4-(trans,trans-4-Pentylbicyclohex-4'-yl)phenyl]-5-pentylpyrimidin
2-[4-(trans,trans-4-Pentylbicyclohex-4'-yl)phenyl]-5-octylpyrimidin
2-[4-(trans,trans-4-Pentylbicyclohex-4'-yl)phenyl]-5-decylpyrimidin
2-[4-(trans,trans-4-Pentylbicyclohex-4'-yl)phenyl]-5-dodecylpyrimidin
2-[4-(trans,trans-4-Pentylbicyclohex-4'-yl)phenyl]-5-ethoxypyrimidin
2-[4-(trans,trans-4-Pentylbicyclohex-4'-yl)phenyl]-5-propoxypyrimidin
2-[4-(trans,trans-4-Pentylbicyclohex-4'-yl)phenyl]-5-butoxypyrimidin
2-[4-(trans,trans-4-Pentylbicyclohex-4'-yl)phenyl]-5-pentoxypyrimidin
2-[4-(trans,trans-4-Pentylbicyclohex-4'-yl)phenyl]-5-hexoxypyrimidin
2-[4-(trans,trans-4-Pentylbicyclohex-4'-yl)phenyl]-5-heptoxypyrimidin
2-[4-(trans,trans-4-Pentylbicyclohex-4'-yl)phenyl]-5-octoxypyrimidin
2-[4-(trans,trans-4-Pentylbicyclohex-4'-yl)phenyl]-5-decoxypyrimidin
2-[4-(trans,trans-4-Pentylbicyclohex-4'-yl)phenyl]-5-dodecoxypyrimidin

**Beispiel 2**

Man erhitzt 2,9 g n-Propylmalondialdehydtetraethylacetal, 4,0 g 4-n-Pentyl-trans,trans,trans-p-tercyclohexyl-4"-carbamidinhydrochlorid und 5 ml DMF 15 Stunden auf 150°. Nach üblicher Aufarbeitung erhält man 2-(4-n-Pentyl-trans,trans,trans-p-tercyclohex-4"-yl)-5-n-propylpyrimidin.

Analog werden hergestellt:

2-(4-n-Pentyl-trans,trans,trans-p-tercyclohex-4"-yl)-5-ethylpyrimidin
2-(4-n-Pentyl-trans,trans,trans-p-tercyclohex-4"-yl)-5-butylpyrimidin
2-(4-n-Pentyl-trans,trans,trans-p-tercyclohex-4"-yl)-5-pentylpyrimidin
2-(4-n-Pentyl-trans,trans,trans-p-tercyclohex-4"-yl)-5-octylpyrimidin
2-(4-n-Pentyl-trans,trans,trans-p-tercyclohex-4"-yl)-5-decylpyrimidin
2-(4-n-Pentyl-trans,trans,trans-p-tercyclohex-4"-yl)-5-dodecylpyrimidin
2-(4-n-Pentyl-trans,trans,trans-p-tercyclohex-4"-yl)-5-ethoxypyrimidin
2-(4-n-Pentyl-trans,trans,trans-p-tercyclohex-4"-yl)-5-propoxypyrimidin
2-(4-n-Pentyl-trans,trans,trans-p-tercyclohex-4"-yl)-5-butoxypyrimidin
2-(4-n-Pentyl-trans,trans,trans-p-tercyclohex-4"-yl)-5-pentoxypyrimidin
2-(4-n-Pentyl-trans,trans,trans-p-tercyclohex-4"-yl)-5-hexoxypyrimidin
2-(4-n-Pentyl-trans,trans,trans-p-tercyclohex-4"-yl)-5-heptoxypyrimidin
2-(4-n-Pentyl-trans,trans,trans-p-tercyclohex-4"-yl)-5-octoxypyrimidin
2-(4-n-Pentyl-trans,trans,trans-p-tercyclohex-4"-yl)-5-decoxypyrimidin
2-(4-n-Pentyl-trans,trans,trans-p-tercyclohex-4"-yl)-5-dodecoxypyrimidin

**Beispiel 3**

Analog zu Beispiel 1 und 2 erhält man aus 4-(trans-4-n-Hexylcyclohexyl)-benzamidinhydrochlorid und trans-4-n-Propylcyclohexylmalondialdehyd-tetramethylacetal (erhältlich aus trans-4-n-Propylcyclohexancarbaldehyd durch Wittig-Reaktion mit Methoxymethyltriphenylphosphoniumchlorid/Kaliumtertiärbutylat und Umsetzung des entstandenen Enolethers mit Orthoameisensäuremethylester/ Bortrifluorid-Etherat) 2-[4-trans-4-n-Hexylcyclohexyl)-phenyl]-5-(trans-4-n-propylcyclohexyl)-pyrimidin.

Analog werden hergestellt:

2-[4-(trans-4-Hexylcyclohexyl)-phenyl]-5-(trans-4-ethyl-cyclohexyl)-pyrimidin
2-[4-(trans-4-Hexylcyclohexyl)-phenyl]-5-(trans-4-butyl-cyclohexyl)-pyrimidin
2-[4-(trans-4-Hexylcyclohexyl)-phenyl]-5-(trans-4-pentyl-cyclohexyl)-pyrimidin
2-[4-(trans-4-Hexylcyclohexyl)-phenyl]-5-(trans-4-heptyl-cyclohexyl)-pyrimidin
2-[4-(trans-4-Hexylcyclohexyl)-phenyl]-5-(trans-4-octyl-cyclohexyl)-pyrimidin
2-[4-(trans-4-Hexylcyclohexyl)-phenyl]-5-(trans-4-nonyl-cyclohexyl)-pyrimidin
2-[4-(trans-4-Hexylcyclohexyl)-phenyl]-5-(trans-4-decyl-cyclohexyl)-pyrimidin

2-[4-(trans-4-Ethylcyclohexyl)-phenyl]-5-(trans-4-ethyl-cyclohexyl)-pyrimidin
2-[4-(trans-4-Ethylcyclohexyl)-phenyl]-5-(trans-4-propyl-cyclohexyl)-pyrimidin
2-[4-(trans-4-Ethylcyclohexyl)-phenyl]-5-(trans-4-butyl-cyclohexyl)-pyrimidin
2-[4-(trans-4-Ethylcyclohexyl)-phenyl]-5-(trans-4-pentyl-cyclohexyl)-pyrimidin
2-[4-(trans-4-Ethylcyclohexyl)-phenyl]-5-(trans-4-heptyl-cyclohexyl)-pyrimidin
2-[4-(trans-4-Ethylcyclohexyl)-phenyl]-5-(trans-4-octyl-cyclohexyl)-pyrimidin
2-[4-(trans-4-Ethylcyclohexyl)-phenyl]-5-(trans-4-nonyl-cyclohexyl)-pyrimidin
2-[4-(trans-4-Ethylcyclohexyl)-phenyl]-5-(trans-4-decyl-cyclohexyl)-pyrimidin

2-[4-(trans-4-Propylcyclohexyl)-phenyl]-5-(trans-4-ethyl-cyclohexyl)-pyrimidin
2-[4-(trans-4-Propylcyclohexyl)-phenyl]-5-(trans-4-propyl-cyclohexyl)-pyrimidin
2-[4-(trans-4-Propylcyclohexyl)-phenyl]-5-(trans-4-butyl-cyclohexyl)-pyrimidin
2-[4-(trans-4-Propylcyclohexyl)-phenyl]-5-(trans-4-pentyl-cyclohexyl)-pyrimidin
2-[4-(trans-4-Propylcyclohexyl)-phenyl]-5-(trans-4-heptyl-cyclohexyl)-pyrimidin
2-[4-(trans-4-Propylcyclohexyl)-phenyl]-5-(trans-4-octyl-cyclohexyl)-pyrimidin
2-[4-(trans-4-Propylcyclohexyl)-phenyl]-5-(trans-4-nonyl-cyclohexyl)-pyrimidin
2-[4-(trans-4-Propylcyclohexyl)-phenyl]-5-(trans-4-decyl-cyclohexyl)-pyrimidin

2-[4-(trans-4-Pentylcyclohexyl)-phenyl]-5-(trans-4-ethyl-cyclohexyl)-pyrimidin
2-[4-(trans-4-Pentylcyclohexyl)-phenyl]-5-(trans-4-propyl-cyclohexyl)-pyrimidin
2-[4-(trans-4-Pentylcyclohexyl)-phenyl]-5-(trans-4-butyl-cyclohexyl)-pyrimidin
2-[4-(trans-4-Pentylcyclohexyl)-phenyl]-5-(trans-4-pentyl-cyclohexyl)-pyrimidin
2-[4-(trans-4-Pentylcyclohexyl)-phenyl]-5-(trans-4-heptyl-cyclohexyl)-pyrimidin
2-[4-(trans-4-Pentylcyclohexyl)-phenyl]-5-(trans-4-octyl-cyclohexyl)-pyrimidin
2-[4-(trans-4-Pentylcyclohexyl)-phenyl]-5-(trans-4-nonyl-cyclohexyl)-pyrimidin
2-[4-(trans-4-Pentylcyclohexyl)-phenyl]-5-(trans-4-decyl-cyclohexyl)-pyrimidin

2-[4-(trans-4-Heptylcyclohexyl)-phenyl]-5-(trans-4-ethyl-cyclohexyl)-pyrimidin
2-[4-(trans-4-Heptylcyclohexyl)-phenyl]-5-(trans-4-propyl-cyclohexyl)-pyrimidin
2-[4-(trans-4-Heptylcyclohexyl)-phenyl]-5-(trans-4-butyl-cyclohexyl)-pyrimidin
2-[4-(trans-4-Heptylcyclohexyl)-phenyl]-5-(trans-4-pentyl-cyclohexyl)-pyrimidin
2-[4-(trans-4-Heptylcyclohexyl)-phenyl]-5-(trans-4-heptyl-cyclohexyl)-pyrimidin
2-[4-(trans-4-Heptylcyclohexyl)-phenyl]-5-(trans-4-octyl-cyclohexyl)-pyrimidin
2-[4-(trans-4-Heptylcyclohexyl)-phenyl]-5-(trans-4-nonyl-cyclohexyl)-pyrimidin
2-[4-(trans-4-Heptylcyclohexyl)-phenyl]-5-(trans-4-decyl-cyclohexyl)-pyrimidin

**Beispiel 4**

Analog zu Beispiel 3 erhält man aus trans,trans-4-n-Heptylbicyclohexyl-4'-carbaldehyd (aus dem entsprechenden Säurechlorid durch Rosenmund-Reduktion) trans,trans-4-n-Heptylbicyclohex-4'-yl-malondialdehydtetramethylacetal und daraus durch Umsetzung mit trans-4-n-Pentylcyclohexancarbamidinhydrochlorid analog zu Beispiel 1 und 2 2-(trans-4-n-Pentylcyclohexyl)-5-(trans,trans-4-n-heptylbicyclohex-4'-yl)-pyrimidin.

Analog werden hergestellt:

2-(trans-4-Pentylcyclohexyl)-5-(trans,trans-4-ethyl-bicyclohex-4'-yl)-pyrimidin
2-(trans-4-Pentylcyclohexyl)-5-(trans,trans-4-propyl-bicyclohex-4'-yl)-pyrimidin
2-(trans-4-Pentylcyclohexyl)-5-(trans,trans-4-butyl-bicyclohex-4'-yl)-pyrimidin
2-(trans-4-Pentylcyclohexyl)-5-(trans,trans-4-pentyl-bicyclohex-4'-yl)-pyrimidin
2-(trans-4-Pentylcyclohexyl)-5-(trans,trans-4-heptyl-bicyclohex-4'-yl)-pyrimidin
2-(trans-4-Pentylcyclohexyl)-5-(trans,trans-4-octyl-bicyclohex-4'-yl)-pyrimidin
2-(trans-4-Pentylcyclohexyl)-5-(trans,trans-4-nonyl-bicyclohex-4'-yl)-pyrimidin
2-(trans-4-Pentylcyclohexyl)-5-(trans,trans-4-decyl-bicyclohex-4'-yl)-pyrimidin

2-(trans-4-Propylcyclohexyl)-5-(trans,trans-4-ethyl-bicyclohex-4'-yl)-pyrimidin
2-(trans-4-Propylcyclohexyl)-5-(trans,trans-4-propyl-bicyclohex-4'-yl)-pyrimidin
2-(trans-4-Propylcyclohexyl)-5-(trans,trans-4-butyl-bicyclohex-4'-yl)-pyrimidin
2-(trans-4-Propylcyclohexyl)-5-(trans,trans-4-pentyl-bicyclohex-4'-yl)-pyrimidin
2-(trans-4-Propylcyclohexyl)-5-(trans,trans-4-heptyl-bicyclohex-4'-yl)-pyrimidin
2-(trans-4-Propylcyclohexyl)-5-(trans,trans-4-octyl-bicyclohex-4'-yl)-pyrimidin
2-(trans-4-Propylcyclohexyl)-5-(trans,trans-4-nonyl-bicyclohex-4'-yl)-pyrimidin
2-(trans-4-Propylcyclohexyl)-5-(trans,trans-4-decyl-bicyclohex-4'-yl)-pyrimidin

2-(trans-4-Butylcyclohexyl)-5-(trans,trans-4-ethyl-bicyclohex-4'-yl)-pyrimidin

2-(trans-4-Butylcyclohexyl)-5-(trans,trans-4-propyl-bicyclohex-4'-yl)-pyrimidin
2-(trans-4-Butylcyclohexyl)-5-(trans,trans-4-butyl-bicyclohex-4'-yl)-pyrimidin
2-(trans-4-Butylcyclohexyl)-5-(trans,trans-4-pentyl-bicyclohex-4'-yl)-pyrimidin
2-(trans-4-Butylcyclohexyl)-5-(trans,trans-4-heptyl-bicyclohex-4'-yl)-pyrimidin
2-(trans-4-Butylcyclohexyl)-5-(trans,trans-4-octyl-bicyclohex-4'-yl)-pyrimidin
2-(trans-4-Butylcyclohexyl)-5-(trans,trans-4-nonyl-bicyclohex-4'-yl)-pyrimidin
2-(trans-4-Butylcyclohexyl)-5-(trans,trans-4-decyl-bicyclohex-4'-yl)-pyrimidin

2-(trans-4-Heptylcyclohexyl)-5-(trans,trans-4-ethyl-bicyclohex-4'-yl)-pyrimidin
2-(trans-4-Heptylcyclohexyl)-5-(trans,trans-4-propyl-bicyclohex-4'-yl)-pyrimidin
2-(trans-4-Heptylcyclohexyl)-5-(trans,trans-4-butyl-bicyclohex-4'-yl)-pyrimidin
2-(trans-4-Heptylcyclohexyl)-5-(trans,trans-4-pentyl-bicyclohex-4'-yl)-pyrimidin
2-(trans-4-Heptylcyclohexyl)-5-(trans,trans-4-heptyl-bicyclohex-4'-yl)-pyrimidin
2-(trans-4-Heptylcyclohexyl)-5-(trans,trans-4-octyl-bicyclohex-4'-yl)-pyrimidin
2-(trans-4-Heptylcyclohexyl)-5-(trans,trans-4-nonyl-bicyclohex-4'-yl)-pyrimidin
2-(trans-4-Heptylcyclohexyl)-5-(trans,trans-4-decyl-bicyclohex-4'-yl)-pyrimidin

**Beispiel 5**

2,8 g trans,trans-4-n-Butylbicyclohexyl-4'-carbonylchlorid, 2,3 g 2-(4-Hydroxyphenyl)-5-n-butylpyrimidin und 0,8 g Pyrimidin werden in 20 ml Toluol 3 Stunden auf 100° erhitzt. Nach dem Abkühlen wird das Pyridinhydrochlorid abgesaugt. Übliche Aufarbeitung liefert 3,2 g 2-[4-(4-trans,trans-n-Butylbicyclohex-4'-yl-carbonyloxy)-phenyl]-5-n-butylpyrimidin.

Analog werden hergestellt:

2-[4-(4-trans,trans-Ethylbicyclohex-4'-ylcarbonyloxy)-phenyl]-5-butylpyrimidin
2-[4-(4-trans,trans-Propylbicyclohex-4'-ylcarbonyloxy)-phenyl]-5-butylpyrimidin
2-[4-(4-trans,trans-Pentylbicyclohex-4'-ylcarbonyloxy)-phenyl]-5-butylpyrimidin
2-[4-(4-trans,trans-Heptylbicyclohex-4'-ylcarbonyloxy)-phenyl]-5-butylpyrimidin
2-[4-(4-trans,trans-Octylbicyclohex-4'-ylcarbonyloxy)-phenyl]-5-butylpyrimidin
2-[4-(4-trans,trans-Nonylbicyclohex-4'-ylcarbonyloxy)-phenyl]-5-butylpyrimidin
2-[4-(4-trans,trans-Decylbicyclohex-4'-ylcarbonyloxy)-phenyl]-5-butylpyrimidin
2-[4-(4-trans,trans-Cyanbicyclohex-4'-ylcarbonyloxy)-phenyl]-5-butylpyrimidin

2-[4-(4-trans,trans-Ethylbicyclohex-4'-ylcarbonyloxy)-phenyl]-5-propylpyrimidin
2-[4-(4-trans,trans-Propylbicyclohex-4'-ylcarbonyloxy)-phenyl]-5-propylpyrimidin
2-[4-(4-trans,trans-Butylbicyclohex-4'-ylcarbonyloxy)-phenyl]-5-propylpyrimidin
2-[4-(4-trans,trans-Pentylbicyclohex-4'-ylcarbonyloxy)-phenyl]-5-propylpyrimidin
2-[4-(4-trans,trans-Heptylbicyclohex-4'-ylcarbonyloxy)-phenyl]-5-propylpyrimidin
2-[4-(4-trans,trans-Octylbicyclohex-4'-ylcarbonyloxy)-phenyl]-5-propylpyrimidin
2-[4-(4-trans,trans-Nonylbicyclohex-4'-ylcarbonyloxy)-phenyl]-5-propylpyrimidin
2-[4-(4-trans,trans-Decylbicyclohex-4'-ylcarbonyloxy)-phenyl]-5-propylpyrimidin

2-[4-(4-trans,trans-Ethylbicyclohex-4'-ylcarbonyloxy)-phenyl]-5-pentylpyrimidin
2-[4-(4-trans,trans-Propylbicyclohex-4'-ylcarbonyloxy)-phenyl]-5-pentylpyrimidin
2-[4-(4-trans,trans-Butylbicyclohex-4'-ylcarbonyloxy)-phenyl]-5-pentylpyrimidin
2-[4-(4-trans,trans-Pentylbicyclohex-4'-ylcarbonyloxy)-phenyl]-5-pentylpyrimidin
2-[4-(4-trans,trans-Heptylbicyclohex-4'-ylcarbonyloxy)-phenyl]-5-pentylpyrimidin
2-[4-(4-trans,trans-Octylbicyclohex-4'-ylcarbonyloxy)-phenyl]-5-pentylpyrimidin
2-[4-(4-trans,trans-Nonylbicyclohex-4'-ylcarbonyloxy)-phenyl]-5-pentylpyrimidin
2-[4-(4-trans,trans-Decylbicyclohex-4'-ylcarbonyloxy)-phenyl]-5-pentylpyrimidin

2-[4-(4-trans,trans-Ethylbicyclohex-4'-ylcarbonyloxy)-phenyl]-5-heptylpyrimidin
2-[4-(4-trans,trans-Propylbicyclohex-4'-ylcarbonyloxy)-phenyl]-5-heptylpyrimidin
2-[4-(4-trans,trans-Butylbicyclohex-4'-ylcarbonyloxy)-phenyl]-5-heptylpyrimidin
2-[4-(4-trans,trans-Pentylbicyclohex-4'-ylcarbonyloxy)-phenyl]-5-heptylpyrimidin
2-[4-(4-trans,trans-Heptylbicyclohex-4'-ylcarbonyloxy)-phenyl]-5-heptylpyrimidin
2-[4-(4-trans,trans-Octylbicyclohex-4'-ylcarbonyloxy)-phenyl]-5-heptylpyrimidin
2-[4-(4-trans,trans-Nonylbicyclohex-4'-ylcarbonyloxy)-phenyl]-5-heptylpyrimidin
2-[4-(4-trans,trans-Decylbicyclohex-4'-ylcarbonyloxy)-phenyl]-5-heptylpyrimidin

2-[4-(4-trans,trans-Ethylbicyclohex-4'-ylcarbonyloxy)-phenyl]-5-nonylpyrimidin
2-[4-(4-trans,trans-Propylbicyclohex-4'-ylcarbonyloxy)-phenyl]-5-nonylpyrimidin
2-[4-(4-trans,trans-Butylbicyclohex-4'-ylcarbonyloxy)-phenyl]-5-nonylpyrimidin

2-[4-(4-trans,trans-Pentylbicyclohex-4'-ylcarbonyloxy)-phenyl]-5-nonylpyrimidin
2-[4-(4-trans,trans-Heptylbicyclohex-4'-ylcarbonyloxy)-phenyl]-5-nonylpyrimidin
2-[4-(4-trans,trans-Octylbicyclohex-4'-ylcarbonyloxy)-phenyl]-5-nonylpyrimidin
2-[4-(4-trans,trans-Nonylbicyclohex-4'-ylcarbonyloxy)-phenyl]-5-nonylpyrimidin
2-[4-(4-trans,trans-Decylbicyclohex-4'-ylcarbonyloxy)-phenyl]-5-nonylpyrimidin

2-[4-(4-trans,trans-Ethylbicyclohex-4'-ylcarbonyloxy)-phenyl]-5-dodecylpyrimidin
2-[4-(4-trans,trans-Propylbicyclohex-4'-ylcarbonyloxy)-phenyl]-5-dodecylpyrimidin
2-[4-(4-trans,trans-Bytylbicyclohex-4'-ylcarbonyloxy)-phenyl]-5-dodecylpyrimidin
2-[4-(4-trans,trans-Pentylbicyclohex-4'-ylcarbonyloxy)-phenyl]-5-dodecylpyrimidin
2-[4-(4-trans,trans-Heptylbicyclohex-4'-ylcarbonyloxy)-phenyl]-5-dodecylpyrimidin
2-[4-(4-trans,trans-Octylbicyclohex-4'-ylcarbonyloxy]-phenyl]-5-dodecylpyrimidin
2-[4-(4-trans,trans-Nonylbicyclohex-4'-ylcarbonyloxy)-phenyl]-5-dodecylpyrimidin
2-[4-(4-trans,trans-Decylbicyclohex-4'-ylcarbonyloxy)-phenyl]-5-dodecylpyrimidin

2-[4-(4-trans,trans-Ethylbicyclohex-4'-ylcarbonyloxy)-phenyl]-5-heptoxypyrimidin
2-[4-(4-trans,trans-Propylbicyclohex-4'-ylcarbonyloxy)-phenyl]-5-heptoxypyrimidin
2-[4-(4-trans,trans-Bytylbicyclohex-4'-ylcarbonyloxy-phenyl]-5-heptoxypyrimidin
2-[4-(4-trans,trans-Pentylbicyclohex-4'-ylcarbonyloxy)-phenyl]-5-heptoxypyrimidin
2-[4-(4-trans,trans-Heptylbicyclohex-4'-ylcarbonyloxy)-phenyl]-5-heptoxypyrimidin
2-[4-(4-trans,trans-Octylbicyclohex-4'-ylcarbonyloxy)-phenyl]-5-heptoxypyrimidin
2-[4-(4-trans,trans-Nonylbicyclohex4'-ylcarbonyloxy)-phenyl]-5-heptoxypyrimidin
2-[4-(4-trans,trans-Decylbicyclohex-4'-ylcarbonyloxy)-phenyl]-5-heptoxypyrimidin

2-[4-(4-trans,trans-Ethylbicyclohex-4'-ylcarbonyloxy)-phenyl]-5-cyanpyrimidin
2-[4-(4-trans,trans-Propylbicyclohex-4'-ylcarbonyloxy)-phenyl]-5-cyanpyrimidin
2-[4-(4-trans,trans-Butylbicyclohex-4'-ylcarbonyloxy)-phenyl]-5-cyanpyrimidin
2-[4-(4-trans,trans-Pentylbicyclohex-4'-ylcarbonyloxy)-phenyl]-5-cyanpyrimidin
2-[4-(4-trans,trans-Heptylbicyclohex-4'-ylcarbonyloxy)-phenyl]-5-cyanpyrimidin
2-[4-(4-trans,trans-Octylbicyclohex-4'-ylcarbonyloxy)-phenyl]-5-cyanpyrimidin
2-[4-(4-trans,trans-Nonylbicyclohex-4'-ylcarbonyloxy)-phenyl]-5-cyanpyrimidin
2-[4-(4-trans,trans-Decylbicyclohex-4'-ylcarbonyloxy)-phenyl]-5-cyanpyrimidin

2-[4-(4-trans-Ethylcyclohexyl-p-phenylcarbonyloxy)-phenyl]-5-pentylpyrimidin
2-[4-(4-trans-Propylcyclohexyl-p-phenylcarbonyloxy)-phenyl]-5-pentylpyrimidin
2-[4-(4-trans-Butylcyclohexyl-p-phenylcarbonyloxy)-phenyl]-5-pentylpyrimidin
2-[4-(4-trans-Pentylcyclohexyl-p-phenylcarbonyloxy)-phenyl]-5-pentylpyrimidin
2-[4-(4-trans-Heptylcyclohexyl-p-phenylcarbonyloxy)-phenyl]-5-pentylpyrimidin
2-[4-(4-trans-Octylcyclohexyl-p-phenylcarbonyloxy)-phenyl]-5-pentylpyrimidin
2-[4-(4-trans-Nonylcyclohexyl-p-phenylcarbonyloxy)-phenyl]-5-pentylpyrimidin
2-[4-(4-trans-Decylcyclohexyl-p-phenylcarbonyloxy)-phenyl]-5-pentylpyrimidin

2-[4-(4-trans-Ethylcyclohexyl-p-phenylcarbonyloxy)-phenyl]-5-cyanpyrimidin
2-[4-(4-trans-Propylcyclohexyl-p-phenylcarbonyloxy)-phenyl]-5-cyanpyrimidin
2-[4-(4-trans-Butylcyclohexyl-p-phenylcarbonyloxy)-phenyl]-5-cyanpyrimidin
2-[4-(4-trans-Pentylcyclohexyl-p-phenylcarbonyloxy)-phenyl]-5-cyanpyrimidin
2-[4-(4-trans-Heptylcyclohexyl-p-phenylcarbonyloxy)-phenyl]-5-cyanpyrimidin
2-[4-(4-trans-Octylcyclohexyl-p-phenylcarbonyloxy)-phenyl]-5-cyanpyrimidin
2-[4-(4-trans-Nonylcyclohexyl-p-phenylcarbonyloxy)-phenyl]-5-cyanpyrimidin
2-[4-(4-trans-Decylcyclohexyl-p-phenylcarbonyloxy)-phenyl]-5-cyanpyrimidin

**Beispiel 6**

Veresterung von 2-(4-Hydroxyphenyl)-5-(trans-4-n-heptylcyclohexyl)-pyrimidin mit trans-4-n-Propylcyclohexancarbonsäurechlorid analog zu Beispiel 5 liefert 2-[4-(trans-4-n-Propylcyclohexylcarbonyloxy)-phenyl]-5(trans-4-n-heptylcyclohexyl)-pyrimidin.

Analog werden hergestellt:

2-[4-(trans-4-Propylcyclohexylcarbonyloxy)-phenyl]-5-(trans-4-ethylcyclohexyl)-pyrimidin
2-[4-(trans-4-Propylcyclohexylcarbonyloxy)-phenyl]-5-(trans-4-propylcyclohexyl)-pyrimidin
2-[4-(trans-4-Propylcyclohexylcarbonyloxy)-phenyl]-5-(trans-4-butylcyclohexyl)-pyrimidin
2-[4-(trans-4-Propylcyclohexylcarbonyloxy)-phenyl]-5-(trans-4-pentylcyclohexyl)-pyrimidin
2-[4-(trans-4-Propylcyclohexylcarbonyloxy)-phenyl]-5-(trans-4-octylcyclohexyl)-pyrimidin

2-[4-(trans-4-Propylcyclohexylcarbonyloxy)-phenyl]-5-(trans-4-nonylcyclohexyl)-pyrimidin
2-[4-(trans-4-Propylcyclohexylcarbonyloxy)-phenyl]-5-(trans-4-decylcyclohexyl)-pyrimidin

2-[4-(trans-4-Butylcyclohexylcarbonyloxy)-phenyl]-5-(trans-4-ethylcyclohexyl)-pyrimidin
2-[4-(trans-4-Butylcyclohexylcarbonyloxy)-phenyl]-5-(trans-4-propylcyclohexyl)-pyrimidin
2-[4-(trans-4-Butylcyclohexylcarbonyloxy)-phenyl]-5-(trans-4-butylcyclohexyl)-pyrimidin
2-[4-(trans-4-Butylcyclohexylcarbonyloxy)-phenyl]-5-(trans-4-pentylcyclohexyl)-pyrimidin
2-[4-(trans-4-Butylcyclohexylcarbonyloxy)-phenyl]-5-(trans-4-heptylcyclohexyl)-pyrimidin
2-[4-(trans-4-Butylcyclohexylcarbonyloxy)-phenyl]-5-(trans-4-octylcyclohexyl)-pyrimidin
2-[4-(trans-4-Butylcyclohexylcarbonyloxy)-phenyl]-5-(trans-4-nonylcyclohexyl)-pyrimidin
2-[4-(trans-4-Butylcyclohexylcarbonyloxy)-phenyl]-5-(trans-4-decylcyclohexyl)-pyrimidin

2-[4-(trans-4-Pentylcyclohexylcarbonyloxy)-phenyl]-5-(trans-4-ethylcyclohexyl)-pyrimidin
2-[4-(trans-4-Pentylcyclohexylcarbonyloxy)-phenyl]-5-(trans-4-propylcyclohexyl)-pyrimidin
2-[4-(trans-4-Pentylcyclohexylcarbonyloxy)-phenyl]-5-(trans-4-butylcyclohexyl)-pyrimidin
2-[4-(trans-4-Pentylcyclohexylcarbonyloxy)-phenyl]-5-(trans-4-pentylcyclohexyl)-pyrimidin
2-[4-(trans-4-Pentylcyclohexylcarbonyloxy)-phenyl]-5-(trans-4-heptylcyclohexyl)-pyrimidin
2-[4-(trans-4-Pentylcyclohexylcarbonyloxy)-phenyl]-5-(trans-4-octylcyclohexyl)-pyrimidin
2-[4-(trans-4-Pentylcyclohexylcarbonyloxy)-phenyl]-5-(trans-4-nonylcyclohexyl)-pyrimidin
2-[4-(trans-4-Pentylcyclohexylcarbonyloxy)-phenyl]-5-(trans-4-decylcyclohexyl)-pyrimidin

2-[4-(trans-4-Heptylcyclohexylcarbonyloxy)-phenyl]-5-(trans-4-ethylcyclohexyl)-pyrimidin
2-[4-(trans-4-Heptylcyclohexylcarbonyloxy)-phenyl]-5-(trans-4-propylcyclohexyl)-pyrimidin
2-[4-(trans-4-Heptylcyclohexylcarbonyloxy)-phenyl]-5-(trans-4-butylcyclohexyl)-pyrimidin
2-[4-(trans-4-Heptylcyclohexylcarbonyloxy)-phenyl]-5-(trans-4-pentylcyclohexyl)-pyrimidin
2-[4-(trans-4-Heptylcyclohexylcarbonyloxy)-phenyl]-5-(trans-4-heptylcyclohexyl)-pyrimidin
2-[4-(trans-4-Heptylcyclohexylcarbonyloxy)-phenyl]-5-(trans-4-octylcyclohexyl)-pyrimidin
2-[4-(trans-4-Heptylcyclohexylcarbonyloxy)-phenyl]-5-(trans-4-nonylcyclohexyl)-pyrimidin
2-[4-(trans-4-Heptylcyclohexylcarbonyloxy)-phenyl]-5-(trans-4-decylcyclohexyl)-pyrimidin

2-[4-(4-Cyanphenylcarbonyloxy)-phenyl]-5-(trans-4-ethylcyclohexyl)-pyrimidin
2-[4-(4-Cyanphenylcarbonyloxy)-phenyl]-5-(trans-4-propylcyclohexyl)-pyrimidin
2-[4-(4-Cyanphenylcarbonyloxy)-phenyl]-5-(trans-4-butylcyclohexyl)-pyrimidin
2-[4-(4-Cyanphenylcarbonyloxy)-phenyl]-5-(trans-4-pentylcyclohexyl)-pyrimidin
2-[4-(4-Cyanphenylcarbonyloxy)-phenyl]-5-(trans-4-heptylcyclohexyl)-pyrimidin
2-[4-(4-Cyanphenylcarbonyloxy)-phenyl]-5-(trans-4-octylcyclohexyl)-pyrimidin
2-[4-(4-Cyanphenylcarbonyloxy)-phenyl]-5-(trans-4-nonylcyclohexyl)-pyrimidin
2-[4-(4-Cyanphenylcarbonyloxy)-phenyl]-5-(trans-4-decylcyclohexyl)-pyrimidin

2-[4-(4-Ethoxyphenylcarbonyloxy)-phenyl]-5-(trans-4-ethylcyclohexyl)-pyrimidin
2-[4-(4-Ethoxyphenylcarbonyloxy)-phenyl]-5-(trans-4-propylcyclohexyl)-pyrimidin
2-[4-(4-Ethoxyphenylcarbonyloxy)-phenyl]-5-(trans-4-butylcyclohexyl)-pyrimidin
2-[4-(4-Ethoxyphenylcarbonyloxy)-phenyl]-5-(trans-4-pentylcyclohexyl)-pyrimidin
2-[4-(4-Ethoxyphenylcarbonyloxy)-phenyl]-5-(trans-4-heptylcyclohexyl)-pyrimidin
2-[4-(4-Ethoxyphenylcarbonyloxy)-phenyl]-5-(trans-4-octylcyclohexyl)-pyrimidin
2-[4-(4-Ethoxyphenylcarbonyloxy)-phenyl]-5-(trans-4-nonylcyclohexyl)-pyrimidin
2-[4-(4-Ethoxyphenylcarbonyloxy)-phenyl]-5-(trans-4-decylcyclohexyl)-pyrimidin

2-[4-(trans-2-Pentyl-1,3-dioxan-5-ylcarbonyloxy)-phenyl]-5-(trans-4-ethylcyclohexyl)-pyrimidin
2-[4-(trans-2-Pentyl-1,3-dioxan-5-ylcarbonyloxy)-phenyl]-5-(trans-4-propylcyclohexyl)-pyrimidin
2-[4-(trans-2-Pentyl-1,3-dioxan-5-ylcarbonyloxy)-phenyl]-5-(trans-4-butylcyclohexyl)-pyrimidin
2-[4-(trans-2-Pentyl-1,3-dioxan-5-ylcarbonyloxy)-phenyl]-5-(trans-4-pentylcyclohexyl)-pyrimidin
2-[4-(trans-2-Pentyl-1,3-dioxan-5-ylcarbonyloxy)-phenyl]-5-(trans-4-heptylcyclohexyl)-pyrimidin
2-[4-(trans-2-Pentyl-1,3-dioxan-5-ylcarbonyloxy)-phenyl]-5-(trans-4-octylcyclohexyl)-pyrimidin
2-[4-(trans-2-Pentyl-1,3-dioxan-5-ylcarbonyloxy)-phenyl]-5-(trans-4-nonylcyclohexyl)-pyrimidin
2-[4-(trans-2-Pentyl-1,3-dioxan-5-ylcarbonyloxy)-phenyl]-5-(trans-4-decylcyclohexyl)-pyrimidin

2-[4-(trans-2-Pentyl-1,3-dithian-5-ylcarbonyloxy)-phenyl]-5-(trans-4-ethylcyclohexyl)-pyrimidin
2-[4-(trans-2-Pentyl-1,3-dithian-5-ylcarbonyloxy)-phenyl]-5-(trans-4-propylcyclohexyl)-pyrimidin
2-[4-(trans-2-Pentyl-1,3-dithian-5-ylcarbonyloxy)-phenyl]-5-(trans-4-butylcyclohexyl)-pyrimidin
2-[4-(trans-2-Pentyl-1,3-dithian-5-ylcarbonyloxy)-phenyl]-5-(trans-4-pentylcyclohexyl)-pyrimidin
2-[4-(trans-2-Pentyl-1,3-dithian-5-ylcarbonyloxy)-phenyl]-5-(trans-4-heptylcyclohexyl)-pyrimidin
2-[4-(trans-2-Pentyl-1,3-dithian-5-ylcarbonyloxy)-phenyl]-5-(trans-4-octylcyclohexyl)-pyrimidin
2-[4-(trans-2-Pentyl-1,3-dithian-5-ylcarbonyloxy)-phenyl]-5-(trans-4-nonylcyclohexyl)-pyrimidin
2-[4-(trans-2-Pentyl-1,3-dithian-5-ylcarbonyloxy)-phenyl]-5-(trans-4-decylcyclohexyl)-pyrimidin

**Beispiel 7**

Veresterung von 2-(4-Hydroxyphenyl)-5-[2-(trans-4-n-pentylcyclohexyl)-ethyl]-pyrimidin [erhältlich durch Kondensation von 4-Hydroxybenzamidin-hydrochlorid mit 2-(trans-4-n-Pentylcyclohexyl)-ethylmalondialdehydtetra-methylacetal [erhältlich aus trans-4-n-Pentylcyclohexyl-propionaldehyd]] mit trans-4-n-Pentylcyclohexancarbon-säurechlorid analog zu Beispiel 5 liefert 2-[4-(trans-4-n-Pentylcyclohexylcarbonyloxy)-phenyl]-5-[2-(trans-4-n-pentylcyclohexyl)-ethyl-pyrimidin.

Analog werden hergestellt:

2-[4-(trans-4-Pentylcyclohexylcarbonyloxy)-phenyl]-5-[2-(trans-4-ethylcyclohexyl)-ethyl ]-pyrimidin
2-[4-(trans-4-Pentylcyclohexylcarbonyloxy)-phenyl]-5-[2-(trans-4-propylcyclohexyl)-ethyl ]-pyrimidin
2-[4-(trans-4-Pentylcyclohexylcarbonyloxy)-phenyl]-5-[2-(trans-4-butylcyclohexyl)-ethyl ]-pyrimidin
2-[4-(trans-4-Pentylcyclohexylcarbonyloxy)-phenyl]-5-[2-(trans-4-heptylcyclohexyl)-ethyl ]-pyrimidin
2-[4-(trans-4-Pentylcyclohexylcarbonyloxy)-phenyl]-5-[2-(trans-4-octylcyclohexyl)-ethyl ]-pyrimidin
2-[4-(trans-4-Pentylcyclohexylcarbonyloxy)-phenyl]-5-[2-(trans-4-nonylcyclohexyl)-ethyl ]-pyrimidin
2-[4-(trans-4-Pentylcyclohexylcarbonyloxy)-phenyl]-5-[2-(trans-4-decylcyclohexyl)-ethyl ]-pyrimidin

**Beispiel 8**

Veretherung von 2-(4-Hydroxyphenyl)-5-[2-(trans-4-n-pentylcyclohexyl)-ethyl]-pyrimidin (vgl. Beispiel 7) mit trans-4-n-Pentylcyclohexylmethylbromid in Dimethylformamid in Gegenwart von Kaliumcarbonat liefert 2-[4-(trans-4-n-Pentylcyclohexylmethoxy)-phenyl]-5-[2-(trans-4-n-pentylcyclohexyl)-ethyl]-pyrimidin.

Analog werden hergestellt:

2-[ 4-(trans-4-Pentylcyclohexylmethoxy)-phenyl]-5-[2-(trans-4-ethylcyclohexyl)-ethyl ]-pyrimidin
2-[ 4-(trans-4-Pentylcyclohexylmethoxy)-phenyl]-5-[2-(trans-4-propylcyclohexyl)-ethyl ]-pyrimidin
2-[ 4-(trans-4-Pentylcyclohexylmethoxy)-phenyl]-5-[2-(trans-4-butylcyclohexyl)-ethyl ]-pyrimidin
2-[ 4-(trans-4-Pentylcyclohexylmethoxy)-phenyl]-5-[2-(trans-4-heptylcyclohexyl)-ethyl ]-pyrimidin
2-[ 4-(trans-4-Pentylcyclohexylmethoxy)-phenyl]-5-[2-(trans-4-octylcyclohexyl)-ethyl ]-pyrimidin
2-[ 4-(trans-4-Pentylcyclohexylmethoxy)-phenyl]-5-[2-(trans-4-nonylcyclohexyl)-ethyl ]-pyrimidin
2-[ 4-(trans-4-Pentylcyclohexylmethoxy)-phenyl]-5-[2-(trans-4-decylcyclohexyl)-ethyl ]-pyrimidin

**Beispiel 9**

Erhitzen von 4-(trans-4-n-Hexylcyclohexyl)-benzamidin-hydrochlorid mit 2-(trans-4-n-Propyl-cyclohexyl)-ethylmalondialdehydtetramethylacetal (erhältlich analog der Pentylverbindung in Beispiel 7) und anschließende übliche Aufarbeitung liefert 2-[4-(trans-n-Hexylcyclohexyl)-phenyl]-5-[2-(trans-4-n-propylcyclohexyl)-ethyl]-pyrimidin.

Analog werden hergestellt:

2-[ 4-(trans-4-Hexylcyclohexyl)-phenyl]-5-[2-(trans-4-ethylcyclohexyl)-ethyl ]-pyrimidin
2-[ 4-(trans-4-Hexylcyclohexyl)-phenyl]-5-[2-(trans-4-butylcyclohexyl)-ethyl ]-pyrimidin
2-[ 4-(trans-4-Hexylcyclohexyl)-phenyl]-5-[2-(trans-4-pentylcyclohexyl)-ethyl ]-pyrimidin
2-[ 4-(trans-4-Hexylcyclohexyl)-phenyl]-5-[2-(trans-4-heptylcyclohexyl)-ethyl ]-pyrimidin
2-[ 4-(trans-4-Hexylcyclohexyl)-phenyl]-5-[2-(trans-4-octylcyclohexyl)-ethyl ]-pyrimidin
2-[ 4-(trans-4-Propylcyclohexyl)-phenyl]-5-[2-(trans-4-ethylcyclohexyl)-ethyl ]-pyrimidin
2-[ 4-(trans-4-Propylcyclohexyl)-phenyl]-5-[2-(trans-4-propylcyclohexyl)-ethyl ]-pyrimidin
2-[ 4-(trans-4-Propylcyclohexyl)-phenyl]-5-[2-(trans-4-butylcyclohexyl)-ethyl ]-pyrimidin
2-[ 4-(trans-4-Propylcyclohexyl)-phenyl]-5-[2-(trans-4-pentylcyclohexyl)-ethyl ]-pyrimidin
2-[ 4-(trans-4-Propylcyclohexyl)-phenyl]-5-[2-(trans-4-heptylcyclohexyl)-ethyl ]-pyrimidin
2-[ 4-(trans-4-Propylcyclohexyl)-phenyl]-5-[2-(trans-4-octylcyclohexyl)-ethyl ]-pyrimidin

**Beispiel 10**

8,8 g 1-(trans-4-Propylcyclohexyl)-4-[4-(trans-4-n-pentylcyclohexyl)-phenyl]-butandion-1,4 (dargestellt durch thiazoliumsalz-katalysierte Addition von trans-4-n-Propylcyclohexancarbaldehyd an die aus 4-(trans-4-n-Pentylcyclohexyl)-acetophenon, Formaldehyd und Dimethylamin erhaltene Mannichbase) und 1,28 ml 80 proz. Hydrazinhydrat werden in 70 ml Eisessig 8 Stunden unter gleichzeitigem Durchleiten von Luft auf 75° erhitzt. Das nach dem Abkühlen ausgefallene Produkt wird aus Ethylacetat umkristallisiert. Man erhält 3,9 g 3-(trans-4-n-Propylcyclohexyl)-6-[4-(trans-4-n-pentylcyclohexyl)-phenyl]-pyridazin.

Analog werden hergestellt:

3-(trans-4-Propylcyclohexyl)-6-[4-(trans-4-ethyl-cyclohexyl)-phenyl]-pyridazin
3-(trans-4-Propylcyclohexyl)-6-[4-(trans-4-propyl-cyclohexyl)-phenyl]-pyridazin
3-(trans-4-Propylcyclohexyl)-6-[4-(trans-4-butyl-cyclohexyl)-phenyl]-pyridazin
3-(trans-4-Propylcyclohexyl)-6-[4-(trans-4-heptyl-cyclohexyl)-phenyl]-pyridazin
3-(trans-4-Propylcyclohexyl)-6-[4-(trans-4-octyl-cyclohexyl)-phenyl]-pyridazin
3-(trans-4-Ethylcyclohexyl)-6-[4-(trans-4-ethyl-cyclohexyl)-phenyl]-pyridazin
3-(trans-4-Ethylcyclohexyl)-6-[4-(trans-4-propyl-cyclohexyl)-phenyl]-pyridazin
3-(trans-4-Ethylcyclohexyl)-6-[4-(trans-4-butyl-cyclohexyl)-phenyl]-pyridazin
3-(trans-4-Ethylcyclohexyl)-6-[4-(trans-4-pentyl-cyclohexyl)-phenyl]-pyridazin
3-(trans-4-Ethylcyclohexyl)-6-[4-(trans-4-heptyl-cyclohexyl)-phenyl]-pyridazin
3-(trans-4-Ethylcyclohexyl)-6-[4-(trans-4-octyl-cyclohexyl)-phenyl]-pyridazin
3-(trans-4-Pentylcyclohexyl)-6-[4-(trans-4-ethyl-cyclohexyl)-phenyl]-pyridazin
3-(trans-4-Pentylcyclohexyl)-6-[4-(trans-4-propyl-cyclohexyl)-phenyl]-pyridazin
3-(trans-4-Pentylcyclohexyl)-6-[4-(trans-4-butyl-cyclohexyl)-phenyl]-pyridazin
3-(trans-4-Pentylcyclohexyl)-6-[4-(trans-4-pentyl-cyclohexyl)-phenyl]-pyridazin
3-(trans-4-Pentylcyclohexyl)-6-[4-(trans-4-heptyl-cyclohexyl)-phenyl]-pyridazin
3-(trans-4-Pentylcyclohexyl)-6-[4-(trans-4-octyl-cyclohexyl)-phenyl]-pyridazin

Die folgenden Beispiele betreffen erfindungsgemäße flüssigkristalline Phasen:

**Beispiel A**

Eine flüssigkristalline Phase aus

4,4 % 4-Ethyl-4'-cyanbiphenyl,
3,8 % 4-Propyl-4'-cyanbiphenyl,
4,3 % 4-Butyl-4'-cyanbiphenyl,
3,5 % 4-Cyan-4'-(trans-4-pentylcyclohexyl)-bi-phenyl,
1,0 % 4-Ethyl-4'-(trans-4-propylcyclohexyl)-bi-phenyl,
7,5 % 4-Ethyl-2'-fluor-4'-(trans-4-pentylcyclohexyl)-biphenyl,
4,0 % p-trans-4-Propylcyclohexyl-benzoesäure-(p-propylphenylester),
3,0 % p-trans-4-Pentylcyclohexyl-benzoesäure-(p-propylphenylester),
10,6 % trans-4-Pentylcyclohexancarbonsäure-(p-pentylphenylester),
6,4 % trans-4-Propylcyclohexancarbonsäure-(p-ethoxyphenylester),
2,5 % 4,4'-Bis-(trans-4-propylcyclohexyl)-bi-phenyl,
2,0 % 4-(trans-4-Pentylcyclohexyl)-4'-(trans-4-propylcyclohexyl)-biphenyl,
0,5 % 2-[4-(trans,trans-4-Pentylbicyclohex-4"-yl)-phenyl]-5-n-heptylpyrimidin,
8,5 % trans-1-p-Ethylphenyl-4-propylcyclohexan,
5,0 % trans-1-p-Ethoxyphenyl-4-propylcyclohexan,
5,5 % 2-p-Hexyloxyphenyl-5-hexylpyrimidin,
5,5 % 2-p-Heptyloxyphenyl-5-hexylpyrimidin,
5,5 % 2-p-Octyloxyphenyl-5-hexylpyrimidin,
5,5 % 2-p-Nonyloxyphenyl-5-hexylpyrimidin,
5,5 % 2-p-Decyloxyphenyl-5-hexylpyrimidin und
5,5 % 2-p-Dodecyloxyphenyl-5-hexylpyrimidin,

hat einen Schmelzpunkt von -12°, einen Klärpunkt von 72° und ist gut geeignet als hoch multiplexierbares Dielektrikum.

**Beispiel B**

Eine flüssigkristalline Phase aus

6,6 % 4-Ethyl-4'-cyanbiphenyl,
5,4 % 4-Propyl-4'-cyanbiphenyl,
4,6 % 4-Butyl-4'-cyanbiphenyl,
11,4 % trans-1-p-Ethylphenyl-4-propylcyclohexan,
6,6 % trans-1-p-Ethoxyphenyl-4-propylcyclohexan,
4,6 % 4-Cyan-4'-(trans-4-pentylcyclohexyl)-bi-phenyl,
1,4 % 4-Ethyl-4'-(trans-4-propylcyclohexyl)-bi-phenyl,
10,0 % 4-Ethyl-2'-fluor-4'-(trans-4-pentylcyclo-hexyl)-biphenyl,

5,4 % p-trans-4-Propylcyclohexyl-benzoesäure-(p-propylphenylester),
4,0 % p-trans-4-Pentylcyclohexyl-benzoesäure-(p-propylphenylester),
12,2 % 2-[trans-4-n-Hexylcyclohexyl)-phenyl]-5-(trans-4-n-propylcyclohexyl)-pyrimidin
17,8 % 2-(trans-4-n-Pentylcyclohexyl)-5-(trans,trans-4-n-heptylbicyclohex-4'-yl)-pyrimidin,
1,6 % 2-p-Hexyloxyphenyl-5-hexylpyrimidin,
1,7 % 2-p-Heptyloxyphenyl-5-hexylpyrimidin,
1,7 % 2-p-Octyloxyphenyl-5-hexylpyrimidin,
1,7 % 2-p-Nonyloxyphenyl-5-hexylpyrimidin,
1,7 % 2-p-Decyloxyphenyl-5-hexylpyrimidin und
1,6 % 2-p-Dodecyloxyphenyl-5-hexylpyrimidin

ist ein hoch multiplexierbares Dielektrikum.

**Beispiel C**

Eine flüssigkristalline Phase aus

5,6 % 2-p-Hexyloxyphenyl-5-hexylpyrimidin,
5,5 % 2-p-Heptyloxyphenyl-5-hexylpyrimidin,
5,6 % 2-p-Octyloxyphenyl-5-hexylpyrimidin,
5,6 % 2-p-Nonyloxyphenyl-5-hexylpyrimidin,
5,5 % 2-p-Decyloxyphenyl-5-hexylpyrimidin,
5,6 % 2-p-Dodecyloxyphenyl-5-hexylpyrimidin,
6,6 % 4-Ethyl-4'-cyanbiphenyl,
5,4 % 4-Propyl-4'-cyanbiphenyl,
4,6 % 4-Butyl-4'-cyanbiphenyl,
11,4 % trans-1-p-Ethylphenyl-4-propylcyclohexan,
6,6 % trans-1-p-Ethoxyphenyl-4-propylcyclohexan,
4,6 % 4-Cyan-4'-(trans-4-pentylcyclohexyl)-biphenyl,
1,4 % 4-Ethyl-4'-(trans-4-propylcyclohexyl)-biphenyl,
10,0 % 4-Ethyl-2'-fluor-4'-(trans-4-pentyl-cyclohexyl)-biphenyl,
5,4 % p-trans-4-Propylcyclohexylbenzoesäure-(p-propylphenylester),
4,0 % p-trans-4-Pentylcyclohexylbenzoesäure-(p-propylphenylester),
2,0 % 2-[ 4-(trans-4-n-Hexylcyclohexyl)-phenyl]-5-[2-(trans-4-n-propylcyclohexyl)-ethyl ]-pyrimidin,
1,7 % 2-[ 4-(4-trans,trans-n-Butylbicyclohex-4'-yl-carbonyloxy)-phenyl ]-5-n-butylpyrimidin,
1,1 % 2-[ 4-(trans-4-n-Propylcyclohexylcarbonyloxy)-phenyl ]-5-(trans-4-n-heptylcyclohexyl)-pyrimidin

hat eine Viskosität von 29. $10^{-3}$ Pa. sec und eine optische Anisotropie von +0,16.

**Beispiel D**

Eine flüssigkristalline Phase aus

7,0 % 2-p-Cyanphenyl-5-ethyl-1,3-dioxan,
8,0 % 2-p-Cyanphenyl-5-propyl-1,3-dioxan,
8,0 % 2-p-Cyanphenyl-5-butyl-1,3-dioxan,
7,0 % 2-p-Cyanphenyl-5-pentyl-1,3-dioxan,
9,0 % 4-Cyan-4'-(trans-4-pentylcyclohexyl)-biphenyl,
11,0 % 4-Ethyl-4'-(trans-4-pentylcyclohexyl)-biphenyl,
7,0 % trans-4-Propylcyclohexancarbonsäure-(p-methoxyphenylester),
7,0 % trans-4-Propylcyclohexancarbonsäure-(p-ethoxyphenylester),
6,0 % trans-4-Butylcyclohexancarbonsäure-(p-methoxyphenylester),
7,5 % 2-[4-trans-4-Pentylcyclohexyl)-phenyl]-5-(trans-4-propylcyclohexyl)-pyrimidin,
9,5 % 2-[4-trans-4-Hexylcyclohexyl)-phenyl]5-(trans-4-propylcyclohexyl)-pyrimidin,
10,5 % 2-(trans-4-n-Pentylcyclohexyl)-5-(trans, -trans-4-heptylbicyclohex-4'-yl)-pyrimidin und
2,5 % 2-[(trans-4-n-Pentylcyclohexyl)-5-(trans,-trans-4-propylbicyclohex-4'-yl)-pyrimidin

ist ein hoch multiplexierbares Dielektrikum.

**Patentansprüche**

1. Verbindungen der Formel I

$R^1$-(A-Z)$_n$-A-$R^2$ I

worin

| | |
|---|---|
| $R^1$ und $R^2$ | jeweils unabhängig voneinander eine Alkylgruppe mit 1 - 15 C-Atomen, worin auch eine oder zwei nicht benachbarte $CH_2$-Gruppen durch O-Atome und/oder -CO-Gruppen und/oder -O-CO-Gruppen und/oder -CO-O-Gruppen ersetzt sein können, einer der Reste $R^1$ und $R^2$ auch H, CN, F, Cl oder Br, |
| die Gruppen A | jeweils unabhängig voneinander eine 1,4-Phenylengruppe, worin auch eine oder zwei CH-Gruppen durch N-Atome ersetzt sein können, eine 1,4-Cyclohexylengruppe, worin auch eine oder zwei nicht benachbarte $CH_2$-Gruppen durch O-Atome ersetzt sein können, eine 1,3-Dithian-2,5-diyl-Gruppe, eine 1,4-Bicyclo(2,2,2)-octylen-Gruppe, eine Decahydronaphthalin-2,6-diyl-Gruppe oder eine 1,2,3,4-Tetrahydronaphthalin-2,6-diylgruppe, |
| die Gruppen Z | jeweils unabhängig voneinander -CO-O-, -O-CO-, $-CH_2CH_2-$, $-OCH_2-$, $-CH_2O-$ oder eine Einfachbindung und |
| n | 3 oder 4 |

bedeutet, mit den Maßgaben, daß

(a) mindestens eine der Gruppen A eine 1,4-Phenylengruppe, worin zwei CH-Gruppen durch N-Atome ersetzt sind, bedeutet, und
(b) mindestens eine der weiteren Gruppen A einen cycloaliphatischen Rest enthält und/oder mindestens eine Gruppe Z nicht eine Einfachbindung ist.

2. Verfahren zur Herstellung von Verbindungen der Formel I nach Anspruch 1, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,

oder daß man eine Verbindung, die sonst der Formel I entspricht, aber an Stelle von C=C-Doppelbindungen eine oder mehrere oxidierbare Gruppen wie $-CH_2CH_2-$, enthält, mit einem oxidierenden Mittel behandelt,

oder daß man zur Herstellung von Estern der Formel I (worin $R_1$ und/oder $R^2$ eine Alkylgruppe bedeuten, worin eine oder zwei $CH_2$-Gruppen durch -O-CO- Gruppen und/oder -CO-O-Gruppen ersetzt sind und/oder worin mindestens eine der Gruppen Z -CO-O- oder -O-CO- bedeutet) eine entsprechende Carbonsäure oder eines ihrer reaktionsfähigen Derivate mit einem entsprechenden Alkohol oder einem seiner reaktionsfähigen Derivate umsetzt,

oder daß man zur Herstellung von 1,3-Dioxanderivaten bzw. 1,3-Dithianderivaten der Formel I (worin $A^1$ 1,3-Dioxan-2,5-diyl bzw. 1,3-Dithian-2,5-diyl bedeutet) einen entsprechenden Aldehyd mit einem entsprechenden Diol bzw. Dithiol umsetzt,

oder daß man zur Herstellung von Ethern der Formel I (worin $R_1$ und/oder $R^2$ eine Alkylgruppe bedeutet, worin eine oder zwei $CH_2$-Gruppen durch O-Atome ersetzt sind und/oder Z eine $-OCH_2-$ oder $-CH_2O$-Gruppe ist) eine entsprechende Hydroxyverbindung verethert,

und/oder daß man gegebenenfalls eine Base der Formel I durch Behandeln mit einer Säure in eines ihrer Säureadditionssalze umwandelt,

oder daß man gegebenenfalls eine Verbindung der Formel I aus einem ihrer Säureadditionssalze durch Behandeln mit einer Base freisetzt.

3. Verwendung der Verbindungen der Formel I nach Anspruch 1 als Komponenten flüssigkristalliner Phasen.

4. Flüssigkristalline Phase mit mindestens zwei flüssigkristallinen Komponenten, dadurch gekennzeichnet, daß mindestens eine Komponente eine Verbindung der Formel I nach Anspruch 1 ist.

5. Flüssigkristallanzeigeelement, dadurch gekennzeichnet, daß es eine Phase nach Anspruch 4 enthält.

6. Elektrooptisches Anzeigeelement nach Anspruch 5, dadurch gekennzeichnet, daß es als Dielektrikum eine Phase nach Anspruch 4 enthält.

**Claims**

1. Compounds of the formula I

$R^1$-(A-Z)$_n$-A-$R^2$ I

wherein

| | |
|---|---|
| $R^1$ and $R^2$ | in each case independently of one another are an alkyl group with 1 - 15 C atoms, wherein one or two non-adjacent $CH_2$ groups can also be replaced by O atoms and/or -CO- groups and/or -O-CO- groups and/or -CO-O- groups, and one of the radicals $R^1$ and $R^2$ can also be H, CN, F, Cl or Br, |
| the groups A | in each case independently of one another are a 1,4-phenylene group, wherein one or two CH groups can also be replaced by N atoms, a 1,4-cyclohexylene group, wherein one or two $CH_2$ groups which are not adjacent can be replaced by O atoms, a 1,3-dithiane-2,5-diyl group, a 1,4-bicyclo(2,2,2)-octylene group, a decahydronapthalene-2,6-diyl group or a 1,2,3,4-tetrahydronaphthalene-2,6-diyl group, |
| the groups Z | in each case independently of one another are -CO-O-, -O-CO-, $-CH_2CH_2-$, $-OCH_2-$, $-CH_2O-$ or a single bond and |
| n | is 3 or 4, |

with the proviso that

(a) at least one of the groups A is a 1,4-phenylene group, wherein two CH groups are replaced by N atoms, and
(b) at least one of the other groups A contains a cycloaliphatic radical and/or at least one group Z is not a single bond.

2. Process for the preparation of compounds of the formula I according to Claim 1, characterised in that a compound which otherwise corresponds to the formula I that contains one or more reducible groups and/or C-C bonds instead of H atoms is treated with a reducing agent,

or in that a compound which otherwise corresponds to the formula I that contains one or more oxidisable groups, such as $-CH_2CH_2-$, instead of C=C-double bonds is treated with an oxidising agent,

or in that, to prepare esters of the formula I (wherein $R^1$ and/or $R^2$ are an alkyl group, wherein one or two $CH_2$ groups are replaced by -O-CO-groups and/or -CO-O groups and/or wherein at least one of the groups Z is -CO-O- or -O-CO-), a corresponding carboxylic acid or one of its reactive derivatives is reacted with a corresponding alcohol or one of its reactive derivatives,

or in that, to prepare 1,3-dioxane derivatives or 1,3-dithiane derivatives of the formula I (wherein $A^1$ is 1,3-dioxane-2,5-diyl or 1,3-dithiane-2,5-diyl), a corresponding aldehyde is reacted with a corresponding diol or dithiol,

or in that, to prepare ethers of the formula I (wherein $R^1$ and/or $R^2$ are an alkyl group, wherein one or two $CH_2$ groups are replaced by O atoms and/or Z is an $-OCH_2-$ or $-CH_2O-$ group), a corresponding hydroxy compound is etherified,

and/or in that, if appropriate, a base of the formula I is converted into one of its acid addition salts by treatment with an acid,

or in that, if appropriate, a compound of the formula I is liberated from one of its acid addition salts by treatment with a base.

3. Use of the compounds of the formula I according to Claim 1 as components of liquid crystal phases.

4. Liquid crystal phase with at least two liquid crystal components, characterised in that at least one component is a compound of the formula I according to Claim 1.

5. Liquid crystal display element, characterised in that it contains a phase according to Claim 4.

6. Electro-optical display element according to Claim 5, characterised in that it contains a phase according to Claim 4 as a dielectric.

**Revendications**

1. Composés de formule I

$R^1$-(A-Z)$_n$-A-$R^2$ I

où

$R^1$ et $R^2$ représentent chacun indépendamment l'un de l'autre un groupe alcoyle en C1 à C15, où également un ou deux groupes $CH_2$ peuvent être remplacés par des atomes d'oxygène et/ou des groupes -CO et/ou des groupes -O-CO et/ou des groupes -CO-O l'un des radicaux $R^1$ et $R^2$ représente également H, CN, F, Cl ou Br,

les groupes A représentent chacun indépendamment l'un de l'autre un groupe 1,4-phénylène, où encore un ou deux groupes CH peuvent être remplacés par des atomes d'azote, un groupe 1,4-cyclohexylène, où encore un ou deux groupes $CH_2$ non voisins peuvent être remplacés par des atomes d'oxygène, un groupe 1,3-dithian-2,5-diyle, un groupe 1,4-bicyclo(2,2,2)-octylène, un groupe décahydronaphtalène-2,6-diyle ou un groupe 1,2,3,4-tétrahydronaphtalène-2,6-diyle,

les groupes Z représentent chacun indépendamment l'un de l'autre -CO-O-, -O-CO-, $-CH_2CH_2-$, $-OCH_2-$, $-CH_2O-$ ou une liaison simple et

n vaut 3 ou 4,

avec ces précisions que

(a) au moins un des groupes A représente un groupe 1,4-phénylène, où deux groupes CH sont remplacés par des atomes d'azote, et
(b) au moins un des autres groupes a contient un radical cycloaliphatique et/ou au moins un groupe Z n'est pas une liaison simple.

2. Procédé de préparation de composés de formule I selon la revendication 1, caractérisé en ce qu'on traite un composé, qui sinon correspond à la formule I, mais contient à la place d'atomes d'hydrogène un ou plusieurs groupes réductibles et/ou liaisons C-C, avec un agent réducteur, ou en ce qu'on traite un composé, qui sinon correspond à la formule I, mais contient à la place de doubles liaisons C=C un ou plusieurs groupes oxydables comme $-CH_2CH_2-$, avec un agent oxydant,

ou en ce que pour préparer des esters de formule I (où $R^1$ et/ou $R^2$ représentent un groupe alcoyle, où un ou deux groupes $CH_2$ sont remplacés par des groupes -O-CO- et/ou des groupes -CO-O- et/ou où au moins l'un des groupes Z représente -CO-O- ou -O-CO-) on fait réagir un acide carboxylique correspondant ou un de ses dérivés réactifs avec un alcool correspondant ou un de ses dérivés réactifs,

ou en ce que pour préparer des dérivés de 1,3-dioxanne ou des dérivés de 1,3-dithiane de formule I (où A1 représente un 1,3-dioxanne-2,5-diyle ou un 1,3-dithiane-2,5-diyle) on fait réagir un aldéhyde correspondant avec un thiol ou dithiol correspondant,

ou en ce que pour préparer des éthers de formule I (où $R^1$ et/ou $R^2$ représentent un groupe alcoyle, où un ou deux groupes $CH_2$ sont remplacés par des atomes d'oxygène et/ou Z est un groupe $-OCH_2-$ ou $-CH_2O-$) on éthérifie un composé hydroxy correspondant,

et/ou en ce qu'on transforme le cas échéant une base de formule I par traitement avec un acide en un de ses sels d'addition d'acides,

ou en ce que le cas échéant on libère un composé de formule I à partir d'un de ses sels d'addition d'acides par traitement avec une base.

3. Application des composés de formule I selon la revendication 1 comme composants de phases à cristaux liquides.

4. Phase cristalline liquide ayant au moins deux composants cristallins liquides, caractérisée en ce qu'au moins un composant est un composé de formule I selon la revendication 1.

5. Elément d'affichage à cristaux liquides, caractérisé en ce qu'il contient une phase selon la revendication 4.

6. Elément d'affichage électro-optique selon la revendication 5, caractérisé en ce qu'il contient comme diélectrique une phase selon la revendication 4.